# EUROPEAN PATENT APPLICATION

(11) **EP 3 925 645 A1**
(43) Date of publication of application: **22.12.2021**
(21) Application number: 20783361.7
(22) Date of filing: 18.02.2020
(51) Int. Cl.: A61M 5/142, A61M 5/145, A61M 5/315

(54) **LIQUID MEDICINE ADMINISTRATION APPARATUS**

(30) Priority: 29.03.2019 JP 2019066302
(71) Applicant: TERUMO Kabushiki Kaisha, Tokyo 151-0072 (JP)
(72) Inventor: HYAKKAN, Yusuke, Ashigarakami-gun, Kanagawa 259-0151 (JP); IWASE, Yoichiro, Ashigarakami-gun, Kanagawa 259-0151 (JP); ARINOBE, Manabu, Ashigarakami-gun, Kanagawa 259-0151 (JP); MAEKAWA, Minami, Ashigarakami-gun, Kanagawa 259-0151 (JP)
(74) Representative: Casalonga
(86) International application number: PCT/JP2020/006283
(87) International publication number: WO 2020/202852

(57) **Abstract**

To provide a liquid medicine administration device capable of preventing leakage of a liquid medicine when blockage occurs in a liquid delivery path of the liquid medicine.

A liquid medicine administration device 100 includes: a liquid medicine container 110 having, at a tip, an opening through which the liquid medicine can be discharged; a gasket 135 for expelling the liquid medicine in the liquid medicine container, the gasket 135 being slidable on the inner wall of the liquid medicine container; a plunger 130 capable of pressing the gasket; a housing 120; a drive mechanism 140 that advances the plunger toward the tip of the liquid medicine container; and a rotation restriction unit 150 that restricts the rotation of the plunger with respect to the housing. The drive mechanism includes: a motor 141; a feed screw 142 that rotates in response to rotation of the motor; and a transmission unit 10 that transmits a drive torque from the motor to the feed screw. The transmission unit includes a canceling unit 20 that cancels the transmission of the drive torque from the motor to the feed screw, when receiving a torque equal to or greater than a preset cancellation torque from the motor.

## Description

### Technical Field

The present invention relates to a liquid medicine administration device.

### Background Art

Conventionally, a syringe pump type liquid medicine administration device has been known which administers a liquid medicine with which a liquid medicine container is filled into a living body by a pressing action of a plunger. The plunger can be configured to, for example, discharge the liquid medicine from the liquid medicine container when pushed into the liquid medicine container by a rotational driving force of a drive mechanism.

For example, Patent Literature 1 discloses a drive mechanism including a motor that generates a drive torque, a gear reduction mechanism that decreases the speed of rotation of the motor, and a feed screw that rotates in response to a torque from the gear reduction mechanism. The plunger is configured as a bearing of the feed screw, and is also configured to advance by rotation of the feed screw.

### Citation List

### Patent Literature

Patent Literature 1: JP 2015-181835 A

### Summary of Invention

### Technical Problem

However, in the liquid medicine administration device as described above, when blockage occurs in a liquid delivery path of the liquid medicine, a load is applied to a region on the upstream side of the blockage portion in the liquid delivery path by the plunger that is going to advance. As a result, the liquid medicine may leak out in the region upstream of the blockage portion in the liquid delivery path.

The present invention has been accomplished in view of the above problems, and an object thereof is to provide a liquid medicine administration device capable of preventing leakage of a liquid medicine when blockage occurs in a liquid delivery path of the liquid medicine.

### Solution to Problem

A liquid medicine administration device according to the present invention includes: a liquid medicine container filled with a liquid medicine and having, at a tip, an opening through which the liquid medicine is discharged; a gasket for expelling the liquid medicine in the liquid medicine container, the gasket being slidable on an inner wall of the liquid medicine container; a plunger capable of pressing the gasket; a housing that holds the liquid medicine container and the plunger; a drive mechanism that advances the plunger toward the tip of the liquid medicine container; and a rotation restriction unit that restricts rotation of the plunger with respect to the housing. The drive mechanism includes: a motor that generates a drive torque; a feed screw that rotates in response to the drive torque; and a transmission unit that transmits the drive torque from the motor to the feed screw, and the plunger includes a threaded portion threadedly engaged with the feed screw. The plunger advances with rotation of the feed screw while the rotation of the plunger with respect to the housing is being restricted by the rotation restriction unit, and the transmission unit includes a canceling unit that cancels the transmission of the drive torque from the motor to the feed screw when receiving a torque equal to or greater than a preset cancellation torque from the motor.

### Advantageous Effects of Invention

According to the liquid medicine administration device of the present invention, it is possible to prevent leakage of the liquid medicine when blockage occurs in a liquid delivery path of the liquid medicine.

### Brief Description of Drawings

Fig. 1 is a side view of a liquid medicine administration system according to one embodiment of the present invention.
Fig. 2 is a diagram schematically illustrating an example of use of the liquid medicine administration system illustrated in Fig. 1.
Fig. 3 is a schematic perspective view of a liquid medicine administration device constituting the liquid medicine administration system illustrated in Fig. 1.
Fig. 4 is an exploded perspective view of the liquid medicine administration device illustrated in Fig. 1.
Fig. 5 is a perspective view illustrating a canceling unit of the liquid medicine administration device illustrated in Fig. 1 and a periphery thereof.
Fig. 6A is a top view illustrating the canceling unit and its periphery illustrated in Fig. 5.
Fig. 6B is a top view illustrating the canceling unit and its periphery illustrated in Fig. 5.
Fig. 7A is a block diagram of a control system of a liquid medicine administration device according to a first modification.
Fig. 7B is a diagram schematically illustrating a rotation detector and the like according to the first modification.
Fig. 8 is a flowchart illustrating operation of a controller according to the first modification.
Fig. 9A is a front view illustrating a canceling unit and its periphery according to a second modification.
Fig. 9B is a front view illustrating the canceling unit and its periphery according to the second modification.

### Description of Embodiments

Embodiments of the present invention will now be described with reference to the accompanying drawings. It should be noted that the following description is not intended to limit the technical scopes and meanings of terms set forth in the claims of the present application. Further, the dimensional ratios in the drawings are exaggerated for convenience of description, and may differ from the actual ratios.

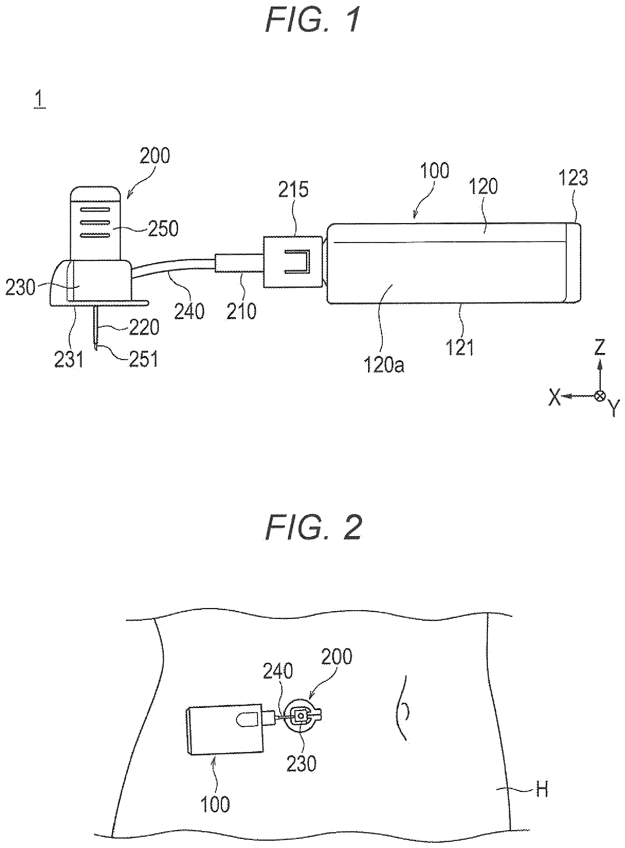

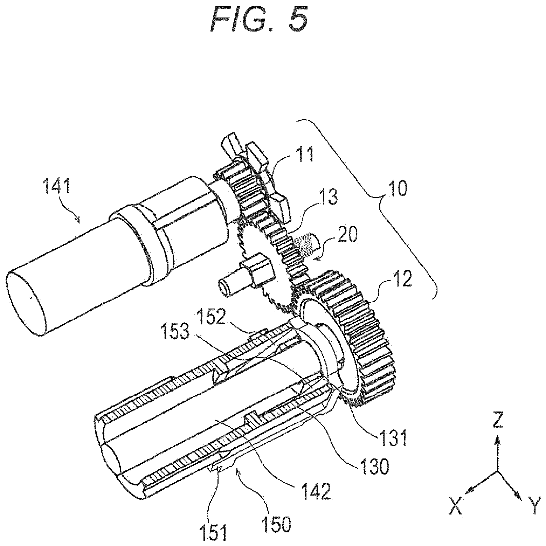

Figs. 1 to 4 are diagrams for describing a liquid medicine administration system 1, a liquid medicine administration device 100, and an administration instrument 200 according to the present embodiment. Figs. 5 to 6B are diagrams for describing a canceling unit 20 according to the present embodiment. In Fig. 5, a plunger 130 is cut out in the axial direction so that the inside of the plunger 130 can be seen. An arrow X in each drawing indicates the "longitudinal direction (longitudinal direction of a liquid medicine container 110)" of the liquid medicine administration device 100, an arrow Y indicates the "width direction (depth direction)" of the liquid medicine administration device 100, and an arrow Z indicates the "height direction" of the liquid medicine administration device 100.

### (Liquid medicine administration system)

The liquid medicine administration system 1 is used to administer a liquid medicine into a living body. As illustrated in Fig. 1, the liquid medicine administration system 1 includes the liquid medicine administration device 100 and the administration instrument 200.

As illustrated in Fig. 2, the liquid medicine administration device 100 and the administration instrument 200 are of a patch type that is attached to the body surface (skin) H of a user when used. The body part of the user to which the liquid medicine administration device 100 and the administration instrument 200 are attached is not particularly limited, and examples thereof include the abdomen and the thigh.

The liquid medicine administration system 1 can continuously administer a liquid medicine (not shown) with which the liquid medicine container 110 is filled included in the liquid medicine administration device 100 into a living body for relatively a long period of time (for example, several minutes to several hours) by a pressing action of a later-described plunger 130. Note that the liquid medicine administration system 1 may administer the liquid medicine at intervals into the living body.

### (Liquid medicine administration device)

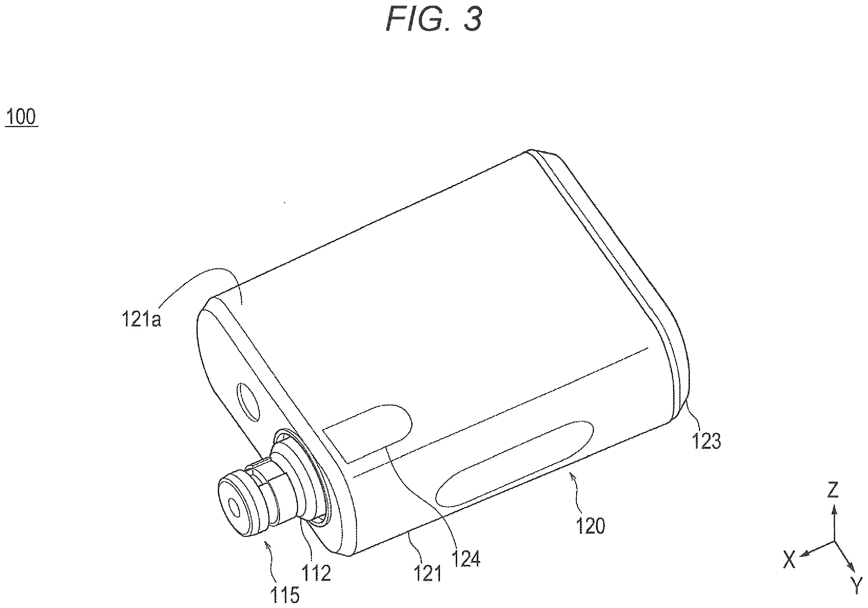

As illustrated in Figs. 3 to 5, the liquid medicine administration device 100 includes: the liquid medicine container 110 filled with a liquid medicine; the plunger 130 that expels the liquid medicine in the liquid medicine container 110; a housing 120 that holds the liquid medicine container 110 and the plunger 130; a drive mechanism 140 that advances the plunger 130 toward a tip of the liquid medicine container 110; a rotation restriction unit 150 that restricts rotation of the plunger 130; a detector 160 that detects a position of the plunger 130; and a controller 170 that controls operations of the drive mechanism 140 and the detector 160.

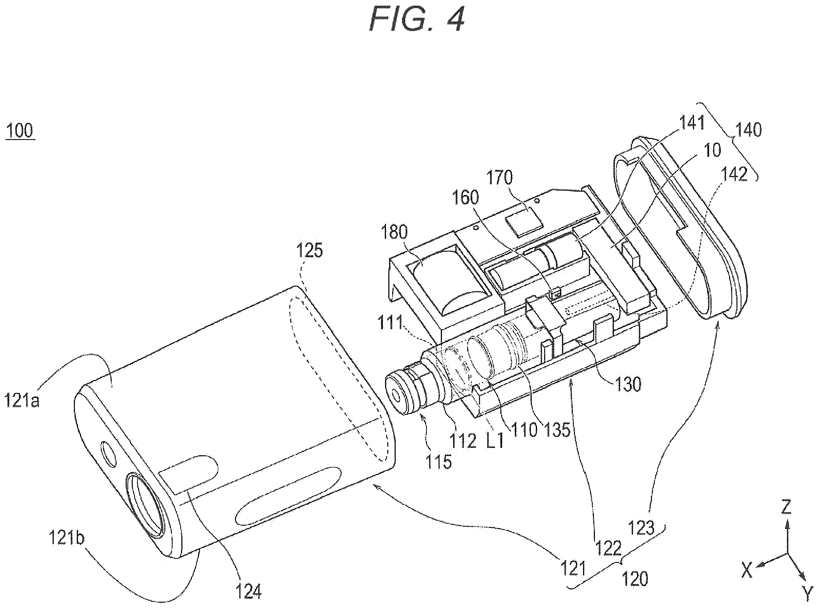

As illustrated in Figs. 3 and 4, the housing 120 has a box-shaped housing body 121 having a housing space formed therein, a chassis 122 that is housed in the housing space of the housing body 121 and can be fixed to the housing body 121, and a lid member 123 attached to the housing body 121 with the chassis 122 being housed in the housing space.

As illustrated in Figs. 3 and 4, a window 124 is formed in an upper surface 121a of the housing body 121 to allow the inside of the housing space to be visible from the outside of the housing 120. The window 124 is formed by providing a transparent or semitransparent portion on a part of the housing body 121.

As illustrated in Figs. 3 and 4, a base end opening 125 through which the chassis 122 is inserted into the housing space of the housing body 121 is formed in the base end of the housing body 121 in the longitudinal direction. The base end opening 125 of the housing body 121 is closed by the lid member 123 with the chassis 122 housed in the housing space.

A bottom surface 121b of the housing body 121 is provided with a sheet-shaped adhesive portion (not shown) that can adhere to the body surface H of the user. In an initial state before the liquid medicine administration device 100 is attached to the user, a releasable protective sheet is attached to an adhesive surface of the adhesive portion.

As illustrated in Fig. 4, the chassis 122 holds the liquid medicine container 110, the plunger 130, the drive mechanism 140, the rotation restriction unit 150, the detector 160, the controller 170, and a power supply unit 180.

The liquid medicine container 110 is a so-called prefilled type liquid medicine container. Therefore, the liquid medicine is charged in advance in a lumen 111 of the liquid medicine container 110. Examples of the liquid medicine include protein preparations, narcotic analgesics, and diuretics.

A sealing member (not shown) for preventing the liquid medicine from leaking is disposed at a tip opening (discharge port) formed at a tip 112 of the liquid medicine container 110. As illustrated in Fig. 3, the tip 112 of the liquid medicine container 110 is disposed to protrude from the housing body 121 to the outside. In addition, a mounting portion 115 that is to be connected to a tube 240 (see Fig. 1) described later is attached to the tip of the liquid medicine container 110 that protrudes from the housing body 121.

As illustrated in Fig. 4, the plunger 130 is inserted into the lumen 111 of the liquid medicine container 110. A gasket 135 slidable on an inner wall of the liquid medicine container 110 is disposed at a tip of the plunger 130. The outer periphery of the gasket 135 is in liquid-tight contact with the inner peripheral surface of the liquid medicine container 110, whereby the base end side of the gasket 135 is liquid-tightly sealed.

The plunger 130 is composed of a cylindrical member. As described above, in Fig. 5, the plunger 130 is cut out in the axial direction so that the inside of the plunger 130 can be seen. The plunger 130 has a threaded portion 131 threadedly engaged with a feed screw 142. The plunger 130 has a notch (not illustrated) on a surface facing the chassis 122, and holds the rotation restriction unit 150 to be described later.

As illustrated in Fig. 4, the drive mechanism 140 includes: a motor 141 that receives a drive current from the power supply unit 180 to generate a rotational driving force; a transmission unit 10 that transmits the rotational driving force of the motor 141; and the feed screw 142 connected to the transmission unit 10.

As illustrated in Fig. 5, the transmission unit 10 includes: a first gear 11 that is connected to the motor 141 and outputs a drive torque of the motor 141; a second gear 12 that is provided on a rotation shaft of the feed screw 142 and rotates together with the feed screw 142; and an intermediate gear 13 that engages with the first gear 11 and the second gear 12 and transmits a torque from the first gear 11 to the second gear 12. Further, the transmission unit 10 includes the canceling unit 20 that releases the engagement between the intermediate gear 13 and the first gear 11 or the second gear 12 when the transmission unit 10 receives a torque equal to or greater than a preset cancellation torque.

In the present specification, the set cancellation torque means a torque having a magnitude received by the transmission unit 10 from the motor 141 when blockage occurs in the liquid delivery path (for example, the tube 240 or the like of the administration instrument 200 to be described later).

In the present embodiment, each of the gears 11, 12, and 13 is constituted by a bevel gear. The intermediate gear 13 is slidably connected to a shaft core, and both ends of the shaft core are fixed to the chassis 122 of the housing 120 (not illustrated).

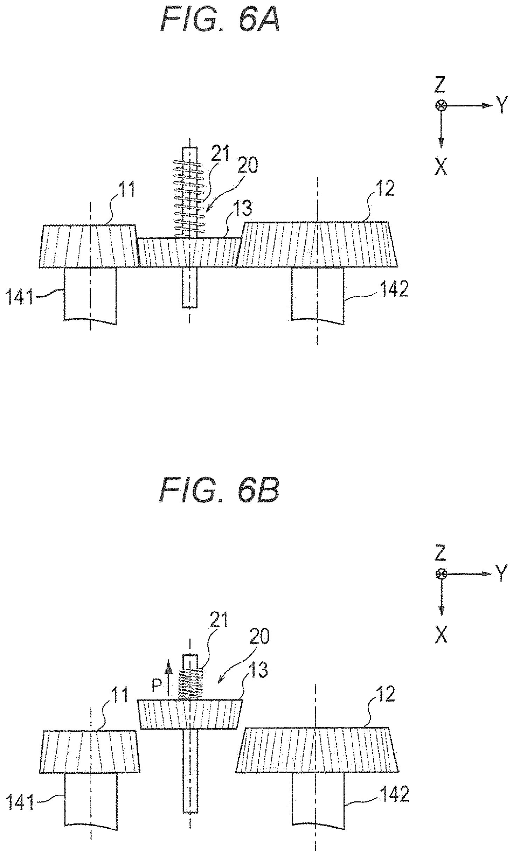

Specifically, engagement surfaces of the intermediate gear 13 and the first gear 11 and engagement surfaces of the intermediate gear 13 and the second gear 12 are inclined (see Figs. 6A and 6B). The engagement surfaces of the gears 11, 12, and 13 which are engaged with each other receive a load in a direction in which they move away from each other according to the torque received when they engage with each other. The shapes of surfaces of the gears 11, 12, and 13 engaged with each other are not particularly limited, and any shapes can be used without departing from the scope thereof.

As illustrated in Fig. 4, the feed screw 142 converts the rotary motion transmitted from the transmission unit 10 into a linear motion, and advances the plunger 130 in the longitudinal direction (X direction).

When the transmission unit 10 receives a torque equal to or greater than the preset cancellation torque from the motor 141, the canceling unit 20 cancels the transmission of the drive torque from the motor 141 to the feed screw 142. The structure of the canceling unit 20 will be described later.

The rotation restriction unit 150 restricts rotation of the plunger 130 with respect to the housing 120. As illustrated in Fig. 5, the rotation restriction unit 150 has a bottom surface 151 fixed to the housing 120 and guide walls 152 and 153 that restrict the rotation of the plunger 130 to urge the plunger 130 to advance in the longitudinal direction. The guide walls 152 and 153 are erected in the height direction (Z direction) from the bottom surface 151. The guide walls 152 and 153 are disposed so as to protrude into the internal space of the plunger 130 from a notch (not illustrated) formed in a surface of the plunger 130 facing the chassis 122. In the present embodiment, the plunger 130 advances in the longitudinal direction with the rotation of the feed screw 142, while the rotation of the plunger 130 with respect to the housing 120 is being restricted by the rotation restriction unit 150. As the plunger 130 advances toward the tip of the liquid medicine container 110, the liquid medicine in the lumen 111 of the liquid medicine container 110 is expelled to the tube 240 (see Fig. 1).

As illustrated in Fig. 4, the detector 160 detects that the plunger 130 has advanced to a predetermined position L1. The predetermined position L1 can be set at any position in the longitudinal direction between a liquid delivery start position of the plunger 130 (the initial position of the plunger 130) to a liquid delivery end position (the position where the plunger 130 completely expels the liquid medicine in the liquid medicine container 110 to the tube 240).

The detector 160 can be constituted by, for example, a known contact sensor that transmits a predetermined electric signal when a detected portion (not illustrated) provided at the base end of the plunger 130 comes into contact therewith.

The controller 170 controls the operation of delivering the liquid medicine by the liquid medicine administration device 100 on the basis of the detection result by the detector 160. The controller 170 can be implemented by, for example, a known microcomputer (electronic circuit element) mounted with a CPU, a RAM, a ROM, and the like. The controller 170 centrally controls operation of the drive mechanism 140, the detector 160, and the power supply unit 180.

In the present embodiment, the controller 170 determines that a predetermined amount of the liquid medicine is not expelled from the liquid medicine container 110 when a predetermined time has elapsed after a command to start the operation is given to the drive mechanism 140, and the detector 160 does not detect the advancement of the plunger 130 to the predetermined position L1. For example, the predetermined time can be set to be longer than a time during which the plunger 130 can sufficiently move to the predetermined position L1 after the controller 170 gives a command to start the operation to the drive mechanism 140 in a case where blockage does not occur in the liquid delivery path of the liquid medicine. When determining that a predetermined amount of the liquid medicine is not expelled, the controller 170 stops the motor 141 and notifies the user of the current situation.

The power supply unit 180 can be composed of, for example, a known battery.

Next, the structure of the canceling unit 20 will be described with reference to Figs. 6A and 6B.

As illustrated in Figs. 6A and 6B, the canceling unit 20 includes a biasing portion 21 that applies a biasing force to the intermediate gear 13.

The biasing portion 21 can be made of, for example, a superelastic alloy such as an alloy of titanium and nickel. The material of the biasing portion 21 is not particularly limited, and a metal material or a resin material may be used as long as it is an elastically deformable elastic member.

In the present embodiment, the biasing portion 21 is formed of a spring member wound around the central axis of the intermediate gear 13. One end of the biasing portion 21 is fixed to the chassis 122 of the housing 120 (not illustrated). The other end of the biasing portion 21 is in contact with the intermediate gear 13.

When the transmission unit 10 receives a torque less than the preset cancellation torque from the motor 141, the biasing portion 21 determines the position of the intermediate gear 13 such that the intermediate gear 13 engages with the first gear 11 and the second gear 12 as illustrated in Fig. 6A. At this time, the intermediate gear 13 receives a force in a direction of compressing the biasing portion 21 from the first gear 11 or the second gear 12.

On the other hand, when blockage occurs in the liquid delivery path of the liquid medicine, the liquid medicine is not delivered, and thus, the plunger 130 receives a drag in a direction opposite to the direction in which the plunger 130 advances. Therefore, the transmission unit 10 including the intermediate gear 13 receives, from the motor 141, a torque greater than that in a case where blockage does not occur.

Therefore, in a case where the transmission unit 10 receives a torque equal to or greater than the preset cancellation torque from the motor 141 due to the occurrence of blockage in the liquid delivery path of the liquid medicine, the force with which the intermediate gear 13 compresses the biasing portion 21 increases, so that the biasing portion 21 is elastically deformed in the compression direction (P direction) as illustrated in Fig. 6B. Then, the biasing portion 21 moves the intermediate gear 13 in a direction in which the intermediate gear 13 is disengaged from the first gear 11 and the second gear 12. As a result, the intermediate gear 13 cannot transmit the torque from the first gear 11 to the second gear 12. At this time, the transmission unit 10 cannot transmit the torque to the feed screw 142 rotating together with the second gear 12, and thus, the feed screw 142 cannot obtain a torque for advancing the plunger 130 in the longitudinal direction. Therefore, when blockage occurs in the liquid delivery path of the liquid medicine, the biasing portion 21 can prevent transmission of the drive torque from the motor 141 to the plunger 130.

### (Administration instrument)

As illustrated in Figs. 1 and 2, the administration instrument 200 is connectable to the liquid medicine administration device 100.

The administration instrument 200 includes a connector 210, a needle tube 220 that is inserted into a living body, a puncture portion (cannula housing) 230, the tube 240, and a puncture assistance tool 250 that assists the puncture of the living body with the needle tube 220.

The connector 210 is connectable to the liquid medicine administration device 100 via a mounting portion 215 fixed to the connector 210. The mounting portion 215 can be connected to the liquid medicine administration device 100 by being externally fitted to the mounting portion 115 (see Fig. 4) which is provided at the tip 112 of the liquid medicine container 110 protruding to the outside of the housing 120.

The mounting portion 215 has inside a connecting needle (not shown) capable of piercing a sealing member (not shown) provided at the tip of the liquid medicine container 110. The tube 240 communicates with the lumen 111 of the liquid medicine container 110 via the connecting needle.

A flow path (not shown) that connects the tube 240 and the lumen of the needle tube 220 is formed inside the puncture portion 230. The liquid medicine delivered to the puncture portion 230 via the tube 240 is administered into the living body through the flow path formed inside the puncture portion 230 and the needle tube 220.

When the liquid medicine is delivered to the user, the puncture assistance tool 250 is attached to the puncture portion 230. The puncture assistance tool 250 holds an introducer needle (inner needle) 251. The introducer needle 251 projects from the tip of the needle tube 220 when the puncture assistance tool 250 is attached to the puncture portion 230. The user can insert the needle tube 220 into the living body, while preventing the needle tube 220 from having any troubles such as breakage, by piercing the living body with the needle tube 220 with the introducer needle 251 inserted into the needle tube 220.

The puncture assistance tool 250 is removed from the puncture portion 230 after the needle tube 220 is inserted into the living body. The introducer needle 251 is withdrawn from the lumen of the needle tube 220 when the puncture assistance tool 250 is removed from the puncture portion 230.

After the needle tube 220 is inserted into the living body, the puncture assistance tool 250 is removed, and the puncture portion 230 is left on the body surface H of the user with the needle tube 220 left in the living body. In this state, the plunger 130 of the liquid medicine administration device 100 advances in the liquid medicine container 110, so that the liquid medicine with which the liquid medicine container 110 is filled is delivered to the lumen of the needle tube 220 through the tube 240 and the flow path of the puncture portion 230.

The introducer needle 251 can be, for example, a metal needle. Further, the needle tube 220 can be composed of, for example, a tubular member (cannula) made of resin.

Similar to the liquid medicine administration device 100, the administration instrument 200 is of a patch type that is attached to the body surface H of the user when used. The contact surface (bottom surface) 231 of the puncture portion 230 of the administration instrument 200 is provided with a sheet-shaped adhesive portion (not shown) that can adhere to the body surface. In an initial state before the administration instrument 200 is attached to the user, a releasable protective sheet is attached to an adhesive surface of the adhesive portion.

As described above, the liquid medicine administration device 100 according to the present embodiment includes: the liquid medicine container 110 filled with a liquid medicine and having, at a tip, an opening through which the liquid medicine can be discharged; the gasket 135 for expelling the liquid medicine in the liquid medicine container 110, the gasket 135 being slidable on the inner wall of the liquid medicine container 110; the plunger 130 capable of pressing the gasket 135; the housing 120 that holds the liquid medicine container 110 and the plunger 130; the drive mechanism 140 that advances the plunger 130 toward the tip of the liquid medicine container 110; and the rotation restriction unit 150 that restricts the rotation of the plunger 130 with respect to the housing 120. The drive mechanism 140 includes: the motor 141, the feed screw 142 that rotates in response to rotation of the motor 141; and the transmission unit 10 that transmits a drive torque from the motor 141 to the feed screw 142. The plunger 130 has the threaded portion 131 threadedly engaged with the feed screw 142, and advances with the rotation of the feed screw 142 while the rotation of the plunger 130 with respect to the housing 120 is being restricted by the rotation restriction unit 150. The transmission unit 10 includes the canceling unit 20 that cancels the transmission of the drive torque from the motor 141 to the feed screw 143, when receiving a torque equal to or greater than a preset cancellation torque from the motor 141.

According to the liquid medicine administration device 100, when blockage occurs in the liquid delivery path of the liquid medicine, the transmission unit 10 receives a torque equal to or greater than the preset cancellation torque from the motor 141. When the transmission unit 10 receives a torque equal to or greater than the preset cancellation torque from the motor 141, the canceling unit 20 cancels the transmission of the drive torque from the motor 141 to the feed screw 142. As a result, the feed screw 142 cannot obtain a torque for advancing the plunger 130 toward the tip of the liquid medicine container 110. Therefore, according to the liquid medicine administration device 100, it is possible to prevent leakage of the liquid medicine when blockage occurs in the liquid delivery path of the liquid medicine.

In addition, the transmission unit 10 includes: the first gear 11 that is connected to the motor 141 and outputs torque; the second gear 12 that is provided on a rotation shaft of the feed screw 142 and rotates together with the feed screw 142; and the intermediate gear 13 that engages with the first gear 11 and the second gear 12 and transmits the torque from the first gear 11 to the second gear 12. The canceling unit 20 disengages the intermediate gear 13 from the first gear 11 or the second gear 12 when the transmission unit 10 receives a torque equal to or greater than the cancellation torque. Therefore, the intermediate gear 13 cannot transmit the torque from the first gear 11 to the second gear 12 by the canceling unit 20. At this time, the transmission unit 10 cannot transmit the torque to the feed screw 142 rotating together with the second gear 12, and thus, the feed screw 142 cannot obtain a torque for advancing the plunger 130 toward the tip of the liquid medicine container 110. Thus, the configuration described above can prevent leakage of the liquid medicine when blockage occurs in the liquid delivery path of the liquid medicine.

The canceling unit 20 is formed of an elastic member. The canceling unit 20 includes a biasing portion 21 that applies a biasing force to the intermediate gear 13 so that the intermediate gear 13 engages with the first gear 11 and the second gear 12. When the intermediate gear 13 receives a torque equal to or greater than the cancellation torque, the biasing portion 21 is elastically deformed in the compression direction. Then, the biasing portion 21 moves the intermediate gear 13 in a direction in which the intermediate gear 13 is disengaged from the first gear 11 or the second gear 12. As a result, the intermediate gear 13 cannot transmit torque from the first gear 11 to the second gear 12. At this time, the transmission unit 10 cannot transmit the torque to the feed screw 142 rotating together with the second gear 12, and thus, the feed screw 142 cannot obtain a torque for advancing the plunger 130 toward the tip of the liquid medicine container 110. Thus, the configuration described above can prevent leakage of the liquid medicine when blockage occurs in the liquid delivery path of the liquid medicine.

The liquid medicine administration device 100 further includes the detector 160 detecting that the plunger 130 has advanced to the predetermined position L1, and the controller 170 that controls the start and stop of the operation of the drive mechanism 140. The controller 170 determines that a predetermined amount of the liquid medicine is not expelled from the liquid medicine container 110, when a predetermined time has elapsed after a command to start the operation is given to the drive mechanism 140, and the detector 160 does not detect that the plunger 130 has advanced to the predetermined position L1. Therefore, when blockage occurs in the liquid delivery path of the liquid medicine, the controller 170 can give a command to stop the operation of the drive mechanism 140 on the basis of the detection result by the detector 160. Thus, the configuration described above can prevent leakage of the liquid medicine when blockage occurs in the liquid delivery path of the liquid medicine.

While the liquid medicine administration device according to the present invention has been described above by way of the embodiment, the present invention is not limited to each of the described configurations, and can be appropriately modified on the basis of the description of the claims. In the following, a modification of the motor will be described. In the description of the modification, the description of the configuration and details of the liquid medicine administration system 1 described above will be appropriately omitted.

### <First Modification>

A liquid medicine administration device 100 according to the first modification uses a DC motor as a motor 141A for the purpose of miniaturization and cost reduction in order to facilitate handling at the time of use and to save a storage space at the time of storage. The DC motor is a coreless motor which is easily downsized and has high torque efficiency with respect to electric power. The DC motor has a characteristic that a current supplied to the DC motor and the rotation speed of the DC motor are different depending on the magnitude of a load torque. The controller 170 detects an administration abnormality (a situation in which a predetermined amount of the liquid medicine is not expelled from the liquid medicine container 110) using the characteristics of the motor 141A. Therefore, a controller 170A controls the drive mechanism 140 as follows.

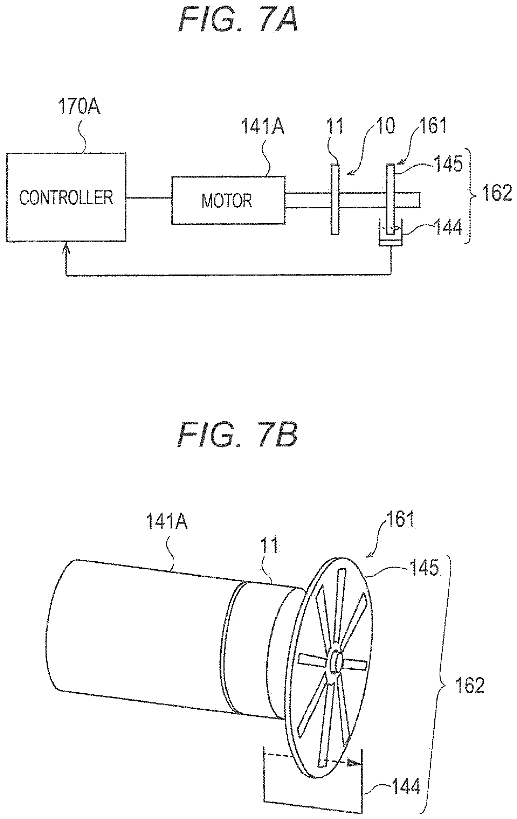

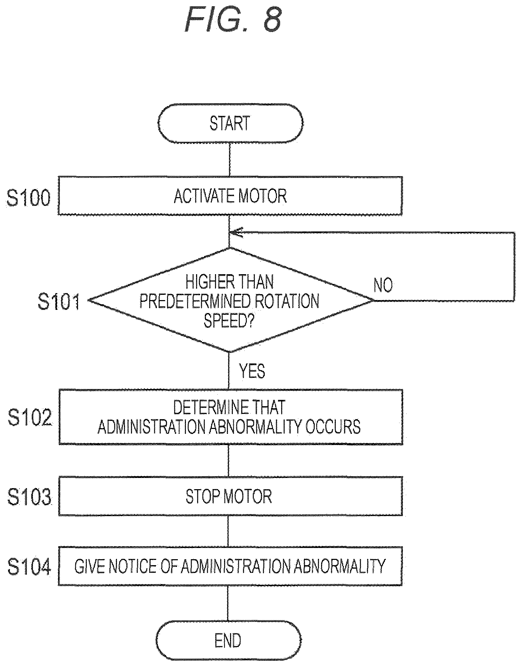

A specific operation of the controller 170A will be described with reference to Figs. 7A, 7B, and 8. Fig. 7A is a block diagram of a control system of the liquid medicine administration device 100 according to the first modification. Fig. 7B is a diagram schematically illustrating a rotation detector 161 and the like according to the first modification. Fig. 8 is a flowchart illustrating operation of the controller 170A according to the first modification.

As illustrated in Fig. 7A, the controller 170A is electrically connected to the motor 141A. A rotation shaft of the motor 141A is mechanically connected to the transmission unit 10. In the transmission unit 10, an encoder 162 is provided as the rotation detector 161 that detects the rotation speed of the motor 141A.

As illustrated in Fig. 7B, the encoder 162 includes a photointerrupter 144 including an optical sensor and a slit plate 145 having a large number of slits which are radially formed. The rotation of the motor 141A is detected by detecting whether or not light passes through the slits of the slit plate 145 with the optical sensor of the photointerrupter 144. The photointerrupter 144 is electrically connected to the controller 170A. In the present embodiment, the encoder 162 using the photointerrupter 144 is described as an example of the rotation detector 161, but an encoder using a magnetic sensor may be used.

When the controller 170A rotates the motor 141A, the transmission unit 10 is driven, so that the plunger 130 advances in the liquid medicine container 110 (see Fig. 4). At this time, the encoder 162 provided adjacent to the transmission unit 10 detects the rotation of the motor 141A, and the controller 170A calculates the rotation speed of the motor 141A on the basis of the rotation of the motor 141A detected by the encoder 162. The rotation of the motor 141A detected by the encoder 162 is fed back to the controller 170A, and the controller 170A calculates the rotation speed of the motor 141A or determines whether or not the motor 141A is rotating on the basis of the feedback result. Note that the rotation detector 161 can also be provided as a part of the transmission unit 10. Specifically, the slit plate 145 is eliminated, and instead, a large number of slits are radially provided in the gear wheel of the first gear 11 of the transmission unit 10. The optical sensor of the photointerrupter 144 detects whether or not light passes through the slits provided in the gear wheel of the first gear 11, whereby the rotation of the motor 141A is detected. In this case, the encoder 162 is constituted by the gear wheel of the first gear 11 of the transmission unit 10 and the photointerrupter 144. With this configuration, the liquid medicine with which the liquid medicine container 110 is filled is delivered to the lumen of the needle tube 220 via the liquid delivery path of the liquid medicine such as the tube 240 and the puncture portion 230, and is administered to the living body.

As illustrated in Fig. 8, the controller 170A activates the motor 141A at the time of administering the liquid medicine to the living body (S100), and determines whether or not the rotation speed of the motor 141A is higher than a predetermined rotation speed (S101). The rotation speed of the motor 141A at the time of administering the liquid medicine to the living body is set in advance according to the rate of administration of the liquid medicine. When the rotation speed of the motor 141A is lower than (not higher than) the predetermined rotation speed (S101: NO), it can be determined that the liquid medicine is normally administered, so that the administration of the liquid medicine is continued. On the other hand, when the rotation speed of the motor 141A is higher than the predetermined rotation speed (exceeds the predetermined rotation speed), it is considered that the gears 11, 12, and 13 of the transmission unit 10 receive torque equal to or greater than the preset cancellation torque from the motor 141A due to an occurrence of abnormality such as blockage of the liquid delivery path of the liquid medicine, resulting in that the intermediate gear 13 is disengaged from the first gear 11 or the second gear 12 by the canceling unit 20 (S101: YES). Therefore, the controller 170A detects an administration abnormality (S102).

Next, the controller 170A stops the motor 141A (S103) and notifies the user of the administration abnormality (S104). The administration abnormality may be notified by lighting or blinking an LED provided on a case of the liquid medicine administration device 100, or by outputting a sound from a speaker provided inside the case of the liquid medicine administration device 100. In addition, the occurrence of the administration abnormality may be wirelessly reported to an external computer.

As described above, in the liquid medicine administration device 100 according to the first modification, the motor 141A is a DC motor. The liquid medicine administration device 100 further includes the rotation detector 161 that detects rotation of the DC motor and the controller 170A that controls rotation of the DC motor. The controller 170A stops the rotation of the motor 141A when the rotation speed of the motor 141A calculated based on the rotation of the motor 141A detected by the rotation detector 161 becomes equal to or higher than a predetermined speed. Therefore, in a case where blockage occurs in the liquid delivery path of the liquid medicine, the controller 170A can prevent the motor 141A from being continuously driven on the basis of the detection result of the detector 160. Thus, the configuration described above can prevent leakage of the liquid medicine when blockage occurs in the liquid delivery path of the liquid medicine.

While an example of the configuration of a medical device has been described above, the present invention is not limited to the description of the above embodiment, and various modifications are possible without departing from the gist of the present invention.

### <Second Modification>

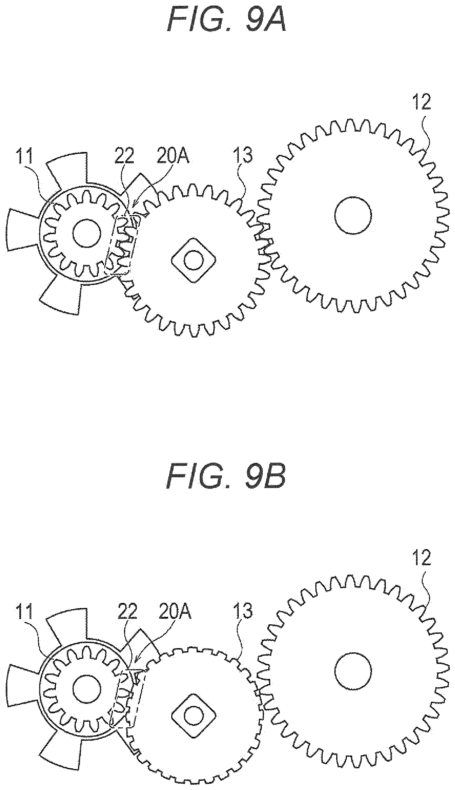

Next, a modification of the canceling unit will be described. A liquid medicine administration device 100 according to the second modification includes a canceling unit 20A having a structure different from that of the canceling unit 20 according to the above embodiment. Figs. 9A and 9B are diagrams for describing the canceling unit according to the second modification.

As illustrated in Fig. 9A, the canceling unit 20A has an engagement tooth portion 22 that is formed on the intermediate gear 13 and that engages with the first gear 11.

The engagement tooth portion 22 is worn by the first gear 11 when the transmission unit 10 receives a torque equal to or greater than a preset cancellation torque from the motor 141. When the engagement tooth portion 22 is worn, the engagement between the intermediate gear 13 and the first gear 11 is released as illustrated in Fig. 9B. As a result, the first gear 11 cannot transmit torque to the intermediate gear 13. Therefore, when blockage occurs in the liquid delivery path of the liquid medicine, the engagement tooth portion 211 can prevent transmission of the drive torque from the motor 141 to the plunger 130.

Note that the material of the engagement tooth portion 22 is not particularly limited, and a metal material or a resin material may be used as long as it is a soft material that wears when receiving a torque equal to or greater than the cancellation torque from the first gear 11.

As described above, the canceling unit 20A according to the second embodiment has the engagement tooth portion 22 that is formed on the intermediate gear 13 and that engages with the first gear 11. When receiving a torque equal to or greater than the cancellation torque from the first gear 11, the engagement tooth portion 22 is worn by the first gear 11, and the engagement between the engagement tooth portion 22 and the first gear 11 is released. Therefore, the engagement tooth portion 22 disables transmission of torque from the first gear 11 to the intermediate gear 13. Thus, the configuration described above can prevent leakage of the liquid medicine when blockage occurs in the liquid delivery path of the liquid medicine.

While the liquid medicine administration device according to the present invention has been described above by way of the modifications, the present invention is not limited to each of the described configurations, and can be appropriately modified on the basis of the description of the claims.

This application is based on Japanese Patent Application No. 2019-066302 filed on March 29, 2019, the disclosure content of which is incorporated by reference in its entirety.

### Reference Signs List

- 1: liquid medicine administration system
- 10: transmission unit
- 11: first gear
- 12: second gear
- 13: intermediate gear
- 20, 20A: canceling unit
- 21: biasing portion
- 22: engagement tooth portion
- 100: liquid medicine administration device
- 110: liquid medicine container
- 120: housing
- 122: chassis
- 130: plunger
- 131: threaded portion
- 135: gasket
- 140: drive mechanism
- 141, 141A: motor
- 142: feed screw
- 144: photointerrupter
- 145: slit plate
- 150: rotation restriction unit
- 160: detector
- 161: rotation detector
- 162: encoder
- 170, 170A: controller
- 180: power supply unit
- 200: administration instrument
- X: longitudinal direction
- L1: predetermined position of plunger
- P: compression direction

## Claims

1. A liquid medicine administration device comprising:
a liquid medicine container filled with a liquid medicine and having, at a tip, an opening through which the liquid medicine is discharged;
a gasket for expelling the liquid medicine in the liquid medicine container, the gasket being slidable on an inner wall of the liquid medicine container;
a plunger capable of pressing the gasket;
a housing that holds the liquid medicine container and the plunger;
a drive mechanism that advances the plunger toward the tip of the liquid medicine container; and
a rotation restriction unit that restricts rotation of the plunger with respect to the housing, wherein
the drive mechanism includes a motor that generates a drive torque, a feed screw that rotates in response to the drive torque, and a transmission unit that transmits the drive torque from the motor to the feed screw,
the plunger has a threaded portion threadedly engaged with the feed screw, and advances with rotation of the feed screw while the rotation of the plunger with respect to the housing is being restricted by the rotation restriction unit, and
the transmission unit includes a canceling unit that cancels the transmission of the drive torque from the motor to the feed screw when receiving a torque equal to or greater than a preset cancellation torque from the motor.

2. The liquid medicine administration device according to claim 1, wherein
the transmission unit includes:
a first gear coupled to the motor and outputting the torque;
a second gear provided on a rotation shaft of the feed screw and rotating together with the feed screw; and
an intermediate gear engaging with the first gear and the second gear and transmitting the torque from the first gear to the second gear, and
the canceling unit releases engagement between the intermediate gear and the first gear or the second gear when the transmission unit receives a torque equal to or greater than the cancellation torque.

3. The liquid medicine administration device according to claim 2, wherein
the canceling unit includes a biasing portion that is formed of an elastic member and that applies a biasing force to the intermediate gear so that the intermediate gear engages with the first gear and the feed screw, and
the biasing portion is elastically deformed in a compression direction to move the intermediate gear in a direction of releasing the engagement between the intermediate gear and the first gear or the second gear, when the transmission unit receives a torque equal to or greater than the cancellation torque.

4. The liquid medicine administration device according to claim 2, wherein
the canceling unit includes an engagement tooth portion that is formed on the intermediate gear and that engages with the first gear, and
when the transmission unit receives a torque equal to or greater than the cancellation torque, the engagement tooth portion is worn by the first gear, and the engagement with the first gear is released.

5. The liquid medicine administration device according to any one of claims 1 to 4, further comprising:
a detector configured to detect that the plunger has advanced to a predetermined position; and
a controller configured to control start and end of operation of the drive mechanism, wherein
the controller determines that a predetermined amount of the liquid medicine has not been expelled from the liquid medicine container, when a predetermined time has elapsed after a command to start the operation is given to the drive mechanism and when the detector does not detect that the plunger has advanced to the predetermined position.

6. The liquid medicine administration device according to any one of claims 1 to 4, wherein
the motor is a DC motor,
the liquid medicine administration device further includes a rotation detector that detects rotation of the DC motor, and a controller that controls the rotation of the DC motor, and
the controller stops the rotation of the DC motor when a rotation speed of the DC motor calculated based on the rotation of the DC motor detected by the rotation detector becomes equal to or higher than a predetermined speed.
